Europäisches Patentamt

European Patent Office

Office européen des brevets

(19)

(11) Numéro de publication : **0 190 518**
**B1**

(12) FASCICULE DE BREVET EUROPÉEN

(45) Date de publication du fascicule du brevet :
17.08.88

(51) Int. Cl.⁴ : **B 01 J 31/22**, C 07 C  9/02,
C 07 C 27/06, C 07 C  1/06//
C07C31/02, C07C29/15

(21) Numéro de dépôt : 85402365.2

(22) Date de dépôt : 02.12.85

(54) Catalyseur d'hydrocondensation du monoxyde de carbone, son procédé de préparation et son application à la fabrication d'hydrocarbures et de composés oxygénés aliphatiques.

(30) Priorité : 05.12.84 FR 8418504

(43) Date de publication de la demande :
13.08.86 Bulletin 86/33

(45) Mention de la délivrance du brevet :
17.08.88 Bulletin 88/33

(84) Etats contractants désignés :
AT BE CH DE FR GB IT LI LU NL SE

(56) Documents cités :
FR-A- 2 374 280
FR-A- 2 496 094
FR-A- 2 516 916
FR-A- 2 563 750
GB-A- 1 193 849
JOURNAL OF THE CHEMICAL SOCIETY, Chemical Communications, no. 19, 1980, pages 908-909; M. BLANCHARD et al.: "Cobalt catalysts for the liquid phase synthesis of light olefins from CO and H2"

(73) Titulaire : NORSOLOR S.A.
Tour Aurore Place des Reflets
F-92080 Paris la Defense Cédex 5 (FR)

(72) Inventeur : Simon, Michel Lucien
35 Rue de Stalingrad
F-62440 Harnes (FR)
Inventeur : Petit, Françis
13 Allée de la Clairière
F-59650 Villeneuve D'Ascq (FR)

(74) Mandataire : Dubost, Thierry Pierre
NORSOLOR Service Propriété Industrielle Tour
Aurore Place des Reflets Cédex 5
F-92080 PARIS LA DEFENSE 2 (FR)

Jouve, 18, rue St-Denis, 75001 Paris, France

## Description

La présente invention concerne un catalyseur d'hydrocondensation du monoxyde de carbone, son procédé de fabrication et son application à la synthèse d'hydrocarbures et de composés oxygénés aliphatiques.

Il est connu, notamment par les brevets français n° 1 583 177, 2 374 280, 2 496 094 et 2 516 916 ainsi que par l'article paru dans Journal of the Chemical Society, Chemical Communications, n° 19 (1980), pages 908-909, de fabriquer un mélange d'hydrocarbures et, le cas échéant, de composés oxygénés à partir de monoxyde de carbone et d'hydrogène en présence d'un catalyseur à base d'un métal choisi parmi le cobalt, le nickel, le ruthénium et le fer. Ces procédés présentent de nombreux inconvénients parmi lesquels :

— un taux de conversion insuffisant, et/ou ;

— de très faibles proportions d'oléfines et de composés oxygénés autres que le méthanol parmi les produits de réaction formés.

Un but de la présente invention consiste en la synthèse d'hydrocarbures et de composés oxygénés aliphatiques à partir de monoxyde de carbone et d'hydrogène avec un taux de conversion élevé et une sélectivité satisfaisante en molécules possédant au moins deux atomes de carbone.

Pour atteindre le but précité, la demanderesse a mis au point un catalyseur d'hydrocondensation du monoxyde de carbone qui constitue un premier objet de la présente invention et qui comprend au moins un produit obtenu par mise en contact de :

(a) au moins un complexe de formule $CuL_n$ dans laquelle n est le nombre de ligands complexant le cuivre et L est un ligand ionique ou covalent,

(b) au moins un activateur de formule $M'R_m$ dans laquelle M' est un métal choisi parmi aluminium, lithium, sodium, magnésium et zinc, m est la valence du métal M' et R est choisi parmi l'atome d'hydrogène et les radicaux hydrocarbonés, le rapport molaire (b)/(a) étant compris entre 0,3 et 20, et

(c) au moins un composé choisi parmi les diènes conjugués, les phosphines, les amines, les arsines et les phosphites, le rapport molaire (c)/(a) étant compris entre 1 et 20.

Ainsi le catalyseur selon l'invention est caractérisé par la mise en contact d'au moins trois composants dans des proportions bien définies. Dans le cadre de cette définition générale, la nature de chaque composant peut être précisée comme suit. Le composant (a) est un complexe ionique ou covalent du cuivre dans lequel les ligands L peuvent être choisis notamment parmi les cétones (en particulier l'acétylacétone) et les anions chlorate, nitrate, carboxylates et halogènes. Le cuivre peut bien entendu être entouré de ligands identiques ou différents. Le composant (b) est un activateur du cuivre, jouant le plus souvent mais non exclusivement le rôle de réducteur et comprenant des liaisons métal-carbone ou métal-hydrogène ; son rapport molaire au composant (a) peut être plus particulièrement compris entre 0,5 et 5. Les radicaux hydrocarbonés liés au métal M' dans le composant (b) peuvent être choisis notamment parmi les radicaux alkyles, cycloalkyles, aryles, arylalkyles et alkylaryles ayant de 1 à 12 atomes de carbone. Des exemples de composants (b) sont en particulier les alkyllithiens RLi, les dialkylzincs $R_2Zn$, les trialkylaluminiums $AlR_3$, l'hydrure double d'aluminium et lithium $LiAlH_4$ et le borohydrure de sodium $NaBH_4$. Les radicaux hydrocarbonés liés au métal M' peuvent bien entendu être identiques ou différents. le composant (c) peut être en particulier le norbornadiène ou le butadiène-1,3 et son rapport molaire au composant (a) peut être plus particulièrement compris entre 3 et 10.

Le catalyseur selon l'invention peut en outre être déposé sur un support polymère organique poreux. Des exemples de polymères adaptés au dépôt d'un tel catalyseur sont notamment des copolymères styrène/divinylbenzène et des copolymères 4-vinylpyridine/divinylbenzène rendus poreux par l'incorporation, au moment de leur fabrication, d'au moins un agent porogène tel que par exemple l'heptane ou l'acide éthyl-2 hexanoïque. De tels copolymères ont en effet une structure poreuse dont la surface spécifique est inférieure ou égale à 250 m²/g et dont le volume poreux est inférieur ou égal à 2 cm³/g. Bien entendu le dépôt du catalyseur selon l'invention sur un tel support ne présente d'intérêt que lorsque la température d'utilisation du catalyseur est inférieure à la température de décomposition du support polymère, celle-ci étant généralement comprise entre 250° et 290 °C pour les copolymères cités précédemment. Le catalyseur de l'invention peut également être déposé sur un support minéral tel que l'alumine, la magnésie, la silice ou incorporé dans une zéolithe suivant les techniques bien connues de l'homme de l'art. Le catalyseur selon l'invention peut comprendre en outre (d) au moins un composé de formule $ML'p$, dans laquelle M est un métal choisi parmi manganèse, zinc et chrome, p est le nombre de ligands complexant M et L' est un ligand ionique ou covalent, de préférence en quantité telle que le rapport molaire (d)/(a) soit au plus égal à 1. Le catalyseur selon l'invention peut comprendre en outre (e) au moins un composé de formule $CoL''q$, dans laquelle q est le nombre de ligands complexant le cobalt et L'' est un ligand ionique ou covalent, en quantité telle que le rapport molaire (e)/(a) soit au plus égal à 1. Les ligands L' et L'' peuvent être choisis notamment parmi les cétones (en particulier l'acétylacétone) et les anions chlorate, nitrate, carboxylates et halogènes.

Un second objet de la présente invention consiste en un procédé de préparation du catalyseur décrit précédemment par mise en contact des composants (a), (b) et (c) et, le cs échéant, (d) et/ou (e) dans au moins un solvant aromatique essentiellement anhydre et au moins partiellement sous atmosphère d'un

2

gaz choisi parmi hydrogène, azote, monoxyde de carbone et gaz rares. La mise en contact des composants peut être effectuée à une température comprise entre 0° et 60 °C et en une ou plusieurs étapes. Lorsque le procédé de préparation est réalisé en une seule étape, il est essentiel que celle-ci soit conduite sous atmosphère de gaz inerte. Lorsque le procédé selon l'invention est réalisé en plusieurs étapes dont l'une est l'étape d'introduction du composant (b), cette étape peut être conduite sous atmosphère d'un gaz réducteur tel que l'hydrogène. Divers solvants aromatiques, tels que notamment benzène ou toluène, peuvent convenir au procédé selon l'invention sous réserve qu'ils soient véritablement anhydres.

Ainsi comme exemple d'un procédé en plusieurs étapes on peut citer un mode de réalistion consistant, dans une première étape, à solubiliser au moins un composant (a) et, le cas échéant, au moins un composant (d) et/ou (e) dans le benzène anhydre, dans une seconde étape à introduire le composant (b) sous atmosphère d'hydrogène et à le laisser réagir avec le(s) composant(s) (a) et le cas échéant, (d) et/ou (e) pendant quelques minutes et dans une troisième étape à introduire le composant (c) solubilisé dans le benzène anhydre.

Enfin un troisième objet de la présente invention consiste en l'application du catalyseur décrit précédemment à la fabrication d'hydrocarbures et de composés oxygénés aliphatiques par réaction entre le monoxyde de carbone et l'hydrogène. Cette réaction peut être effectuée selon un rapport molaire $CO/H_2$ compris entre 0,1 et 2, sous une pression comprise entre 5 et 400 bars, la température de réaction étant comprise entre 210° et 400 °C, le temps de séjour du mélange réactionnel étant compris entre 0,1 et 120 secondes. La réaction peut être effectuée en présence d'au moins un solvant ayant un point d'ébullition supérieur ou égal à la température de réaction. De préférence un tel solvant n'est ni hydrogénable ni hydrogénolysable dans les conditions de ladite réaction et peut être choisi par exemple parmi le biphényle, l'ortho-terphényle, la décaline, la tétraline et le tétraglyme. Lorsque le catalyseur est isolé à l'état solide ou déposé sur un support, la réaction peut aussi être effectuée selon les principes de la catalyse hétérogène en phase gazeuse en mettant en œuvre soit une technologie en lit fixe, soit une technologie en lit fluidisé soit une technologie en lit entraîné bien connues de l'homme de l'art.

Le procédé de synthèse selon l'invention permet d'obtenir, à partir de monoxyde de carbone et d'hydrogène, des mélanges d'alcanes, d'oléfines et de composés oxygénés ayant de 1 à 8 atomes de carbone.

L'essentiel des composés oxygénés est constitué d'alcools linéaires ayant de 1 à 4 atomes de carbone, tels que méthanol, éthanol, n-propanol et n-butanol, les autres composés oxygénés éventuellement présents des aldéhydes, des cétones, des alcools ramifiés et des esters. La constitution de ces mélanges dépend bien entendu des conditions de synthèse, en particulier la pression, la température, le temps de séjour du mélange réactionnel et la formule chimique du catalyseur.

Les exemples ci-après sont donnés à titre illustratif et non limitatif de la présente invention.

## Exemple 1

Dans un tube de Schlenk on introduit, sous agitation et sous atmosphère d'azote, 6,1 g d'acétylacétonate de cuivre ainsi que 4,0 g d'acétylacétonate de chrome que l'on solubilise dans 70 ml de benzène anhydre. Le rapport molaire Cu/Cr est donc égal à 2. On introduit ensuite 33 ml d'une solution à 1,25 mole par litre de diéthylzinc dans le cyclohexane sous atmosphère d'hydrogène. Après un temps de 5 minutes on ajoute 15 g de butadiène-1,3 solubilisé dans 15 ml de benzène anhydre à 0 °C.

## Exemple 2

A la solution de catalyseur dans le benzène préparée conformément à l'exemple 1 on ajoute 30 g d'ortho-terphényle puis on introduit la solution dans un réacteur autoclave de volume 0,1 litre et la distillation discontinue du benzène est conduite en présence du gaz de synthèse constitué d'un mélange de monoxyde de carbone et d'hydrogène. La réaction d'hydrocondensation est ainsi effectuée avec un rapport molaire $CO/H_2$ égal à 0,5 sous une pression de 80 bars, à la température de 260 °C et avec un temps de séjour du mélange réactionnel égal à 20 secondes. En sortie de réacteur, après détente des gaz, une série de chromatographes en phase gazeuse permet d'analyser et d'identifier les différents produits de synthèse. On trouvera dans le tableau ci-après :

— le taux de conversion T.C. égal au nombre de moles de CO consommées sur le nombre de moles de CO introduites ;

— la sélectivité en hydrocarbures H.C., égale au rapport du nombre de moles de CO transformées en hydrocarbures au nombre de moles de CO transformées, exprimée en pourcent (la conversion de CO en $CO_2$ n'est pas prise en compte) ;

— la sélectivité en produits oxygénés P.O., égale au rapport du nombre de moles de CO transformées en produits oxygénés au nombre de moles de CO transformées (la conversion de CO en $CO_2$ n'est pas prise en compte) ;

— la caractéristique des coupes d'hydrocarbures exprimées en pourcentage molaire ;

— la caractéristique des coupes de composés oxygénés exprimée en pourcentage molaire.

### Exemple 3

L'hydrocondensation du monoxyde de carbone dans un rapport $CO/H_2$ égal à 1 est effectuée dans les conditions de l'exemple 2 à l'exception de la température, égale à 300 °C, et du temps de séjour, égal à 4,6 secondes. En sortie de réacteur, les différents produits de synthèse sont analysés comme précédemment et les résultats figurent dans le tableau ci-après.

### Exemple 4

Dans un tube de Schlenk on introduit, sous agitation et sous atmosphère d'azote, 10,1 g d'acétylacétonate de cuivre que l'on solubilise dans 45 ml de benzène anhydre. On introduit ensuite 31 ml d'une solution à 1,25 mole par litre de diéthylzinc dans le cyclohexane sous atmosphère d'hydrogène. Après un temps de 5 minutes on ajoute 17 g de butadiène-1,3 solubilisé dans 15 ml de benzène anhydre à 0 °C. A la solution de catalyseur ainsi préparée on ajoute 30 g d'ortho-terphényle puis on introduit la solution dans un réacteur autoclave de volume 0,1 litre et la distillation discontinue du benzène est conduite en présence du gaz de synthèse constitué d'un mélange équimoléculaire de monoxyde de carbone et d'hydrogène. La réaction d'hydrocondensation est ainsi effectuée sous une pression de 60 bars, à la température de 260 °C et avec un temps de séjour du mélange réactionnel égal à 18,6 secondes. En sortie de réacteur, après détente des gaz, les différents produits de synthèse sont analysés comme indiqué à l'exemple 2. Les résultats figurent dans le tableau ci-après.

### Exemple 5

Dans un tube de Schlenk on introduit, sous agitation et sous atmosphère d'azote, 2,1 g d'acétylacétonate de cuivre et 2,06 g d'acétylacétonate de cobalt que l'on solubilise dans 100 ml de benzène anhydre. Le rapport molaire Cu/Co est donc égal à 1. On introduit ensuite 14,5 ml d'une solution à 1,25 mole par litre de diéthylzinc dns le cyclohexane sous atmosphère d'hydrogène. Après un temps de 5 minutes on ajoute 6,7 g de butadiène-1,3 solubilisé dans 15 ml de benzène anhydre à 0 °C. A la solution de catalyseur ainsi préparée on ajoute 30 g d'ortho-terphényle puis on introduit la solution dans un réacteur autoclave de volume 0,1 litre et la distillation discontinue du benzène est conduite en présence du gaz de synthèse constitué d'un mélange de monoxyde de carbone et d'hydrogène. La réaction d'hydrocondensation est ainsi effectuée, avec un rapport molaire $CO/H_2$ égal à 0,5 sous une pression de 40 bars, à la température de 280 °C et avec un temps de séjour du mélange réactionnel égal à 17,9 secondes. En sortie de réacteur, après détente des gaz, les produits de synthèse sont analysés comme indiqué à l'exemple 2. Les résultats figurent dans le tableau ci-après.

### Exemple 6

Dans un réacteur autoclave de volume 1 litre on introduit, sous agitation et sous atmosphère d'azote, 12,4 g d'acétylacétonate de cuivre et 6,1 g d'acétylacétonate de cobalt que l'on solubilise dans 360 ml de benzène anhydre. Le rapport molaire Cu/Co est donc égal à 2. On introduit ensuite 56 ml d'une solution à 1,25 mole par litre de diéthylzinc dans l'heptane sous atmosphère d'hydrogène. Après un temps de 5 minutes on ajoute 48,6 g de butadiène-1,3 solubilisé dans 75 ml de benzène anhydre à 0 °C. A la solution de catalyseur ainsi préparée on ajoute 300 g d'ortho-terphényle puis la distillation discontinue du benzène est conduite en présence du gaz de synthèse constitué d'un mélange de monoxyde de carbone et d'hydrogène. La réaction d'hydrocondensation est ainsi effectuée, avec un rapport molaire $CO/H_2$ égal à 0,5 sous une pression de 80 bars, à la température de 300 °C et avec un temps de séjour du mélange réactionnel égal à 37 secondes. En sortie de réacteur, après détente des gaz, les produits de synthèse sont analysés comme indiqué à l'exemple 2. Les résultats figurent dans le tableau ci-après.

### Exemple 7

Dans un tube de Schlenk n° 1 on introduit 2,7 g d'acétylacétonate de cuivre, 3,5 g d'acétylacétonate de chrome et 1,3 g d'acétylacétonate de cobalt que l'on dégaze par vide. Le mélange et conservé sous azote. Le rapport molaire Cu/Cr est égal à 1, le rapport molaire Cu/Co est égal à 2. Dans un tube de Schlenk n° 2 on introduit 24 ml d'une solution à 1,25 mole par litre de diéthylzinc dans l'heptane sous atmosphère d'azote. On ajoute ensuite l'heptane sous atmosphère d'azote. On ajoute ensuite 50 ml de toluène préalablement dégazé, puis la poudre du tube de Schlenk n° 1 est introduite par transfert dans le tube de Schlenk n° 2, cette opération étant effectuée sous atmosphère d'hydrogène. Après un temps de 5 minutes on ajoute 10 g de butadiène-1,3 solubilisé dans 15 ml de toluène anhydre à 0 °C. A la solution de catalyseur ainsi préparée on ajoute 90 g d'ortho-terphényle puis on introduit la solution dans un réacteur autoclave de volume 0,3 litre et la distillation discontinue du toluène et de l'heptane est conduite en présence d'hydrogène jusqu'à 260 °C. La réaction d'hydrocondensation est ensuite effectuée avec un rapport molaire $CO/H_2$ égal à 0,5 sous une pression de 60 bars, à la température de 260 °C et avec un temps de séjour du mélange réactionnel égal à 14,3 secondes. En sortie de réacteur, après détente des gaz, les produits de synthèse sont analysés comme indiqué à l'exemple 2. Les résultats figurent dans le tableau ci-après.

## Exemple 8

Dans un tube de Schlenk on introduit, sous agitation et sous atmosphère d'azote, 10,2 g d'acétylacétonate de cuivre que l'on solubilise dans 100 ml de toluène anhydre. On introduit ensuite 13 ml d'une solution à 2 moles par litre de triéthylaluminium dans le toluène sous atmosphère d'hydrogène. Après un temps de 5 minutes on ajoute 14,2 g de butadiène-1,3 solubilisé dans 30 ml de toluène anhydre à 0 °C. A la solution de catalyseur ainsi préparée on ajoute 300 g d'ortho-terphényle puis on introduit la solution dans un réacteur autoclave de volume 1 litre et la distillation discontinue du toluène est conduite en présence du gaz de synthèse constitué d'un mélange de monoxyde de carbone et d'hydrogène. La réaction d'hydrocondensation est ainsi effectuée avec un rapport molaire $CO/H_2$ égal à 0,5 sous une pression de 61 bars, à la température de 300 °C et avec un temps de séjour du mélange réactionnel égal à 18 secondes. En sortie de réacteur, après détente des gaz, les différents produits de synthèse sont analysés comme indiqué à l'exemple 2. Les résultats figurent dans le tableau ci-après.

## Exemple 9

Dans un tube de Schlenk on introduit, sous agitation et sous atmosphère d'azote, 13,1 g d'acétylacétonate de cuivre, 11,1 g d'acétylacétonate de zinc et 12,8 g d'acétylacétonate de cobalt que l'on solubilise dans 720 ml de benzène anhydre. Le rapport molaire Cu/Co et égal à 1, le rapport molaire Zn/Cu égal à 0,8. On introduit ensuite 125 ml d'une solution à 1,16 mole par litre de triéthylaluminium dans le benzène sous atmosphère d'hydrogène. Après un temps de 5 minutes on ajoute 40 g de butadiène-1,3 solubilisé dans 107 ml de benzène anhydre à 0 °C. A la solution de catalyseur ainsi préparée on ajoute 300 g d'ortho-terphényle puis on introduit la solution dans un réacteur autoclave de volume 1 litre et la distillation discontinue du benzène est conduite en présence du gaz de synthèse constitué d'un mélange de monoxyde de carbone et d'hydrogène. La réaction d'hydrocondensation est ainsi effectuée avec un rapport molaire $CO/H_2$ égal à 0,5 sous une pression de 41 bars, à la température de 260 °C et avec un temps de séjour du mélange réactionnel égal à 31 secondes. En sortie de réacteur, après détente des gaz, les produits de synthèse sont analysés comme indiqué à l'exemple 2. Les résultats figurent dans le tableau ci-après.

(Voir tableau page 6)

**Tableau**

| Exemple | 2 | 3 | 4 | 5 | 6 | 7 | 8 | 9 |
|---|---|---|---|---|---|---|---|---|
| T.C.(%) | 3,3 | 3,4 | 2,2 | 24,9 | 3,7 | 33,0 | 0,4 | 14,7 |
| H.C. | 60,9 | 32,2 | 7,8 | 79,1 | 83,2 | 92,2 | 73,3 | 87,2 |
| P.O. | 39,1 | 67,8 | 92,2 | 20,9 | 16,8 | 7,8 | 26,7 | 12,8 |
| méthane | 65,1 | 77,3 | 68,6 | 69,9 | 85,7 | 84,8 | 73,8 | 81,3 |
| éthane | 21,3 | 11,9 | 6,9 | 11,4 | 10,6 | 8,3 | 16,9 | 4,7 |
| propane | 2,9 | 1,8 | 15,2 | 3,1 | 1,8 | 2,4 | 4,6 | 3,2 |
| butane | 1,2 | 0,5 | 1,8 | 1,5 | 0,2 | 0,9 | - | 2,1 |
| alcanes $C_5$-$C_8$ | 1,0 | 0,2 | 0,5 | 0,6 | - | 0,6 | 1,6 | 3,1 |
| éthylène | 3,3 | 4,8 | 2,6 | 3,5 | 0,5 | 0,4 | - | 0,2 |
| propène | 3,7 | 2,2 | 3,3 | 7,0 | 1,0 | 1,6 | 3,1 | 2,9 |
| butène-1 | 0,9 | 0,4 | 1,1 | 2,0 | 0,2 | 0,5 | - | 1,1 |
| butène-2 | 0,4 | 0,7 | - | 0,3 | - | 0,3 | - | 0,3 |
| alcènes $C_5$-$C_8$ | 0,2 | 0,2 | - | 0,7 | - | 0,2 | - | 1,1 |
| méthanol | 49,4 | 98,8 | 31,9 | 39,8 | 81,0 | 54,9 | 60,9 | 48,6 |
| éthanol | 47,4 | 0,9 | 45,7 | 36,8 | 16,3 | 30,4 | 29,7 | 19,5 |
| n-propanol | - | 0,1 | - | 10,8 | 0,8 | 8,2 | 0,8 | 4,1 |
| n-butanol | - | - | - | 3,9 | - | 2,7 | 0,9 | 1,6 |
| alcools ramifiés | 3,2 | 0,2 | 0,3 | 1,3 | - | 2,3 | - | 1,2 |
| acétates | - | - | 20,9 | 0,5 | 1,7 | 0,1 | 2,5 | 20,9 |
| acétaldéhyde | - | - | 1,2 | 2,8 | - | 0,5 | 1,1 | 1,3 |
| divers | - | - | - | 4,1 | 0,2 | 0,9 | 4,1 | 2,8 |

**Revendications**

1. Catalyseur d'hydrocondensation du monoxyde de carbone caractérisé en ce qu'il comprend au moins un produit obtenu par mise en contact de :

(a) au moins un complexe de formule $CuL_n$ dans laquelle n est le nombre de ligands complexant le cuivre et L est un ligand ionique ou covalent,

(b) au moins un activateur de formule $M'R_m$ dans laquelle M' est un métal choisi parmi aluminium, lithium, sodium, magnésium et zinc, m est la valence du métal M' et R est choisi parmi l'atome d'hydrogène et les radicaux hydrocarbonés, le rapport molaire (b)/(a) étant compris entre 0,3 et 20, et

(c) au moins un composé choisi parmi les diènes conjugués, les phosphines, les amines, les arsines et les phosphites, le rapport molaire (c)/(a) étant compris entre 1 et 20.

2. Catalyseur selon la revendication 1, caractérisé en ce que le ligand L est choisi parmi les cétones et les anions chlorate, nitrate, carboxylates et halogènes.

3. Catalyseur selon l'une des revendications 1 et 2, caractérisé en ce que le composant (c) est le butadiène-1,3.

4. Catalyseur selon l'une des revendications 1 à 3, caractérisé en ce qu'il comprend en outre (d) au moins un composé de formule $ML'_p$ dans laquelle M est un métal choisi parmi manganèse, zinc et chrome, p est le nombre de ligands complexant M et L' est un ligand ionique ou covalent.

5. Catalyseur selon l'une des revendications 1 à 4, caractérisé en ce qu'il comprend en outre (e) au moins un composé de formule $CoL''q$, dans laquelle q est le nombre de ligands complexant le cobalt et L'' est un ligand ionique ou covalent, en quantité telle quel le rapport molaire (e)/(a) soit au plus égal à 1.

6. Catalyseur selon la revendication 4, caractérisé en ce que le rapport molaire (d)/(a) est au plus égal à 1.

7. Procédé de préparation d'un catalyseur selon l'une des revendications 1 à 6, caractérisé en ce qu'il comprend la mise en contact des composants (a), (b) et (c) et, le cas échéant, (d) et/ou (e) dans au moins un solvant aromatique essentiellement anhydre et au moins partiellement sous atmosphère d'un gaz choisi parmi hydrogène, azote, monoxyde de carbone et gaz rares.

8. Procédé selon la revendication 7, caractérisé en ce que la mise en contact des composants est effectuée à une température comprise entre 0 ° et 60 °C.

9. Procédé selon l'une des revendications 7 et 8, caractérisé en ce qu'il est réalisé en plusieurs étapes dont une étape d'introduction du composant (b), cette étape conduite sous atmosphère d'un gaz réducteur.

10. Application d'un catalyseur selon l'une des revendications 1 à 6 à la fabrication d'hydrocarbures et de composés oxygénés aliphatiques par réaction entre le monoxyde de carbone et l'hydrogène dans un rapport molaire $CO/H_2$ compris entre 0,1 et 2, sous une pression comprise entre 5 et 400 bars, à une température comprise entre 210 ° et 400 °C, le temps de séjour du mélange réactionnel étant compris entre 0,1 et 120 secondes et la réaction étant effectuée en présence d'au moins un solvant ayant un point d'ébullition supérieur ou égal à la température de réaction.

11. Application selon la revendication 10, caractérisée en ce que le solvant n'est ni hydrogénable ni hydrogénolysable dans les conditions de la réaction.

12. Application selon l'une des revendications 10 et 11, caractérisée en ce que le solvant est choisi parmi le biphényle, l'orthoterphényle, la décaline, la tétraline et le tétraglyme.

13. Catalyseur selon l'une des revendications 1 à 6, caractérisé en ce qu'il est déposé sur un support polymère organique poreux.

14. Catalyseur selon la revendication 13, caractérisé en ce que ledit support est choisi parmi les copolymères styrène/divinylbenzène poreux et les copolymères 4-vinylpyridine/divinylbenzène poreux.

15. Catalyseur selon l'une des revendications 1 à 6, caractérisé en ce qu'il est déposé sur un support minéral.

16. Catalyseur selon l'une des revendications 1 à 6, caractérisé en ce qu'il est incorporé dans une zéolithe.


**Claims**

1. Catalyst for the hydrocondensation of carbon monoxide, characterized in that it comprises at least one product obtained by bringing together :

(a) at least one complex of formula $CuL_n$ in which n is the number of ligands complexing the copper and L is an ionic or covalent ligand.

(b) at least one activator of formula $M'R_m$ in which M' is a metal chosen from aluminium, lithium, sodium, magnesium and zinc, m is the valency of the metal M' and R is chosen from the hydrogen atom and hydrocarbon radicals, the molar ration (b)/(a) being between 0.3 and 20, and

(c) at least one compound chosen from conjugated dienes, phosphines, amines, arsines and phosphites, the molar ratio (c)/(a) being between 1 and 20.

2. Catalyst according to Claim 1, characterized in that the ligand L is chosen from ketones and the chlorate, nitrate, carboxylate and halogen anions.

3. Catalyst according to either of Claims 1 and 2, characterized in that the component (c) is 1,3-butadiene.

4. Catalyst according to one of Claims 1 to 3, characterized in that it additionally comprises (d) at least one compound of formula $ML'_p$ in which M is a metal chosen from manganese, zinc and chromium, p is the number of ligands complexing M and L' is an ionic or covalent ligand.

5. Catalyst according to one of Claims 1 to 4, characterized in that it additionally comprises (e) at least one compound of formula $CoL''_q$, in which q is the number of ligands complexing the cobalt and L'' is an ionic or covalent ligand, in such quantity that the molar ratio (e)/(a) does not exceed 1.

6. Catalyst according to Claim 4, characterized in that the molar ratio (d)/(a) does not exceed 1.

7. Process for preparing a catalyst according to one of Claims 1 to 6, characterized in that it comprises bringing together the components (a), (b) and (c) and, where appropriate, (d) and/or (e) in at least one essentially anhydrous aromatic solvent and at least partly under an atmosphere of a gas chosen from hydrogen, nitrogen, carbon monoxide and the rare gases.

8. Process according to Claim 7, characterized in that the bringing together of the components is carried out at a temperature of between 0 ° and 60 °C.

9. Process according to either of Claims 7 and 8, characterized in that it is carried out in several stages including a stage for the introduction of the component (b), this stage being carried out under an atmosphere of a reductive gas.

10. Application of a catalyst according to one of Claims 1 to 6 to the manufacture of hydrocarbons and of aliphatic oxygenated compounds by reaction between carbon monoxide and hydrogen in a molar ratio $CO/H_2$ of between 0.1 and 2, at a pressure of between 5 and 400 bars, at a temperature of between 210 ° and 400 °C, the residence time of the reaction mixture being between 0.1 and 120 seconds and the reaction being carried out in the presence of at least one solvent which has a boiling point above or equal to the reaction temperature.

11. Application according to Claim 10, characterized in that the solvent is not capable of being hydrogenated or hydrogenolysed under the reaction conditions.

12. Application according to either of Claims 10 and 11, characterized in that the solvent is chosen from biphenyl, ortho-terphenyl, decalin, tetralin and tetraglyme.

13. Catalyst according to one of Claims 1 to 6, characterized in that it is deposited onto a porous organic polymer support.

14. Catalyst according to Claim 13, characterized in that the said support is chosen from porous styrene/divinylbenzene copolymers and porous 4-vinylpyridine/divinylbenzene copolymers.

15. Catalyst according to one of Claims 1 to 6, characterized in that it is deposited onto an inorganic support.

16. Catalyst according to one of Claims 1 to 6, characterized in that it is incorporated in a zeolite.

## Patentansprüche

1. Kohlenmonoxidhydrokondensationskatalysator, dadurch gekennzeichnet, daß er wenigstens ein Produkt enthält, das erhalten wurde durch Inkontaktbringen von

(a) mindestens einem Komplex der Formel $CuL_n$, in der n die Anzahl der dem Kupferkomplex bildenden Liganden und L ein ionischer oder kovalenter Ligand ist,

(b) mindestens einem Aktivator der Formel $M'R_m$, in der M' ein Metall ausgewählt aus der Gruppe bestehend aus Aluminium, Lithium, Natrium, Magnesium und Zink ist, m die Wertigkeit des Metalls M' ist und R ausgewählt ist aus der Gruppe bestehend aus dem Wasserstoffatom und den Kohlenwasserstoffradikalen, wobei das Molverhältnis (b)/(a) zwischen 0,3 und 20 beträgt, und

(c) mindestens einer Verbindung augewählt aus der Gruppe bestehend aus den konjugierten Dienen, den Phosphinen, den Aminen, den Arsenwasserstoffen und den Phosphiten, wobei das Molverhältnis (c)/(a) zwischen 1 und 20 beträgt.

2. Katalysator nach Anspruch 1, dadurch gekennzeichnet, daß der Ligand L ausgewählt ist aus der Gruppe bestehend aus den Ketonen und den Chlorat-, Nitrat-, Carboxylat- und Halogenanionen.

3. Katalysator nach Anspruch 1 oder 2, dadurch gekennzeichnet, daß die Verbindung (c) Butadien-1,3 ist.

4. Katalysator nach einem der Ansprüche 1 bis 3, dadurch gekennzeichnet, daß er weiters (d) mindestens eine Verbindung der Formel $ML'_p$ enthält, in der M ein Metall ausgewählt aus der Gruppe bestehend aus Mangan, Zink und Chrom ist, p die Anzahl der den M-Komplex bildenden Liganden bedeutet und ein ionischer oder kovalenter Ligand ist.

5. Katalysator nach einem der Ansprüche 1 bis 4, dadurch gekennzeichnet, daß er weiters (e) mindestens eine Verbindung der Formel $CoL''_q$, in der q die Anzahl der den Kobaltkomplex bildenden Liganden bedeutet und L'' ein ionischer oder kovalenter Ligand ist, in einer solchen Menge enthält, daß das Molverhältnis (e)/(a) höchstens 1 ist.

6. Katalysator nach Anspruch 4, dadurch gekennzeichnet, daß das Molverhältnis (d)/(a) höchstens 1 ist.

7. Verfahren zur Herstellung eines Katalysators nach einem der Ansprüche 1 bis 6, gekennzeichnet durch das Inkontaktbringen der Komponenten (a), (b) und (c) und gegebenenfalls (d) und/oder (e) in mindestens einem im wesentlichen wasserfreien aromatischen Lösungsmittel und wenigstens teilweise in einer Gasatmosphäre, wobei das Gas ausgewählt ist aus der Gruppe bestehend aus Wasserstoff, Stickstoff, Kohlenmonoxid und den Edelgasen.

8. Verfahren nach Anspruch 7, dadurch gekennzeichnet, daß das Inkontaktbringen der Komponenten bei einer Temperatur zwischen 0° und 60 °C erfolgt.

9. Verfahren nach Anspruch 7 oder 8, dadurch gekennzeichnet, daß es in mehreren Schritten durchgeführt wird, von denen einer das Einbringen der Komponente (b) ist, wobei dieser Schritt unter reduzierender Gasatmosphäre durchgeführt wird.

10. Verwendung eines Katalysators nach einem der Ansprüche 1 bis 6 zur Herstellung von Kohlenwasserstoffen und oxygenierten aliphatischen Verbindungen durch Reaktion zwischen dem Kohlenmonoxid und dem Wasserstoff in einem Molverhältnis $CO/H_2$ zwischen 0.1 und 2, unter einem Druck zwischen 5 und 400 bar und bei einer Temperatur zwischen 210° und 400 °C, wobei die Verweilzeit des Reaktionsgemisches zwischen 0,1 und 120 s beträgt und die Reaktion in Gegenwart mindestens eines Lösungsmittels erfolgt, das eine Siedetemperatur besitzt die höher als die oder gleich der Reaktionstemperatur ist.

11. Verwendung nach Anspruch 10, dadurch gekennzeichnet, daß das Lösungsmittel unter den Reaktionsbedingungen weder hydrierbar noch hydrogenolysierbar ist.

12. Verwendung nach Anspruch 10 oder 11, dadurch gekennzeichnet, daß das Lösungsmittel ausgewählt ist aus der Gruppe bestehend aus Biphenyl, Orthoterphenyl, Decalin, Tetralin und Tetraglym.

13. Katalysator nach einem der Ansprüche 1 bis 6, dadurch gekennzeichnet, daß er sich auf einem porösen organischen Polymerträger befindet.

14. Katalysator nach Anspruch 13, dadurch gekennzeichnet, daß der genannte Träger ausgewählt ist aus der Gruppe bestehend aus den porösen Styrol/Divinylbenzol-Copolymeren und den porösen 4-Vinylpyridin/-Divinylbenzol-Copolymeren.

15. Katalysator nach einem der Ansprüche 1 bis 6, dadurch gekennzeichnet, daß er sich auf einen mineralischen Träger befindet.

16. Katalysator nach einem der Ansprüche 1 bis 6, dadurch gekennzeichnet, daß er in einem Zeolith inkorporiert ist.